# EUROPEAN PATENT SPECIFICATION

(11) **EP 1 694 242 B1**
(45) Date of publication and mention of the grant of the patent: **16.04.2014**
(21) Application number: 04800502.9
(22) Date of filing: 01.11.2004
(51) Int. Cl.: A61K 9/08, A61K 47/10, A61K 47/12, A61K 47/18, A61K 47/22, A61K 47/26, A61K 31/4745

(54) **PROCESS FOR MAKING PHARMACEUTICAL 7-(2-TRIMETHYLSILYLETHYL) CAMPTOTHECIN FORMULATIONS**
VERFAHREN ZUR HERSTELLUNG VON PHARMAZEUTISCHEN 7-(2-TRIMETHYLSILYLETHYL) CAMPTOTHECIN FORMULIERUNGEN
PROCEDE PERMETTANT D'OBTENIR DES PREPARATIONS PHARMACEUTIQUES DE 7-(2-TRIMETHYLSILYLETHYL) CAMPTOTHECIN

(30) Priority: 17.12.2003 US 530154 P
(43) Date of publication of application: 30.08.2006
(73) Proprietor: BIONUMERIK PHARMACEUTICALS, INC., San Antonio, Texas 78229 (US)
(72) Inventor: HAUSHEER, Frederick, H., Fair Oaks Ranch, TX 78015 (US); WANG, Jianyan, Helotes, TX 78023 (US); KOCHAT, Harry, San Antonio, TX 78248-2208 (US)
(74) Representative: Lucas, Phillip Brian
(86) International application number: PCT/US2004/036191
(87) International publication number: WO 2005/060871

(56) References cited:
- WO-A1-98/35940
- US-A- 5 726 181
- US-A- 5 958 937
- US-A- 5 958 937
- ANNEMARIE H. VAN HATTUM ET AL: "New highly lipophilic camptothecin BNP1350 is an effective drug in experimental human cancer", INTERNATIONAL JOURNAL OF CANCER, vol. 88, no. 2, 15 October 2000 (2000-10-15), pages 260-266, XP55007980, ISSN: 0020-7136, DOI: 10.1002/1097-0215(20001015)88:2<260::AID-I JC18>3.0.CO;2-Q
- PATRICK A. THOMPSON ET AL: "Plasma and cerebrospinal fluid pharmacokinetic study of BNP1350 in nonhuman primates", CANCER CHEMOTHERAPY AND PHARMACOLOGY, vol. 53, no. 6, 1 June 2004 (2004-06-01), pages 527-532, XP55007979, ISSN: 0344-5704, DOI: 10.1007/s00280-004-0765-6

## Description

### FIELD OF THE INVENTION

This application relates to pharmaceutical formulations and will have particular application to formulations of highly lipophilic camptothecin compounds adapted for intravenous administration to human patients undergoing treatment for cancer and other diseases.

### BACKGROUND OF THE INVENTION

Camptothecin (CPT) and certain of its derivatives are potent antineoplastic agents that are currently the subject of numerous ongoing scientific investigations. Recently, the Untied States Food and Drug Administration approved the first two CPT derivatives (Irinotecan and Topotecan, discussed below) for human use as therapy for various forms of solid neoplasms.

Camptothecin was first isolated in 1966 by Wall and Wani from *Camptotheca accuminata,* a Chinese yew. CPT was subsequently observed to have potent anti-cancer activity and was introduced into human clinical trials in the late 1970's. The closed E-ring lactone form of CPT was noted to be very poorly water soluble (approximately 0.1 microgram of drug dissolving in 1 mL of water). In order for CPT to be administered in human clinical trials it was first formulated with sodium hydroxide. This formulation resulted in hydrolysis of the lactone E-ring of the camptothecin molecule and formed the water soluble carboxylate species. The sodium hydroxide formulation of CPT created a water soluble CPT species that permitted clinicians to administer larger doses of the drug to cancer patients undergoing Phase I and Phase II clinical trials. It was not learned until much later that the carboxylate form of CPT had approximately one-tenth or less of the antitumor potency of the lactone form of CPT. Clinical trials with sodium hydroxide formulated CPT were disappointing due to the frequently observed significant systemic toxicities and the lack of antineoplastic activity, and clinical studies of CPT were halted in the early 1980's.

Further clinical development of CPT derivatives was not pursued until the mid-1980s. At that time it was reported that CPT had a unique mechanism of action involving the inhibition of DNA synthesis and DNA replication by interactions with the ubiquitous cellular enzyme Topoisomerase I (Topo I). This new information about the mechanism of action of CPT derivatives rekindled the interest in developing new Topo I inhibitors as antineoplastic drugs and subsequently several research groups began attempting to develop new CPT derivatives for cancer therapy. In general, it was observed that, like CPT, many of its derivatives were also very poorly soluble in water (less than 1 µg/mL). This low water solubility greatly limited the practical clinical utility of the drug because prohibitively large volumes, of fluid had to be administered to the patient in order to provide an effective dose of the drug. Because of the potent antineoplastic activity and poor water solubility of CPT and many of its derivatives in water, a great deal of research effort was directed at generating new CPT derivatives that were water soluble. This research is discussed below.

As stated earlier, CPT and many of its derivatives (Wall and Wani Camptothecin and Taxol: Discovery to Clinic-Thirteenth Bruce F. Cain Memorial Award Lecture Cancer Research 55:753-760; 1995) are poorly water soluble and are reportedly poorly soluble in a number of pharmaceutically acceptable organic solvents as well. There are numerous reports of newly created water soluble derivatives of CPT (Sawada, S. et al; Kingsbury, W. D. et al., Luzzio et al. Synthesis and Antitumor Activity ofNovel Water Soluble Derivatives of Camptothecin as Specific Inhibitors of Topoisomerase I Jour. Med. Chem. 38:395-401; 1995) which have been synthesized in an attempt to overcome some of the significant technical problems in drug administration of poorly water soluble camptothecins to patients with cancer. Several water soluble CPT derivatives have been synthesized in an attempt to address the poor water solubility and difficulties in administration to patients. Well known examples of these water soluble CPT derivatives include: 9-dimethylaminomethyl-10-hydroxy camptothecin (Topotecan), 7-[(4-methylpiperazino)methyl]-10,11-ethylenedioxy camptothecin, 7-[(4-methylpiperazino)methyl]-10,11-methylenedioxy camptothecin, and 7-ethyl-10-[4-(1-piperidino)-1-piperidino]carbonyloxy camptothecin (Irinotecan or CPT-11).

Other substituted CPT derivatives with different solubility and pharmacologic properties have been synthesized as well; examples of these camptothecin derivatives include 9-amino camptothecin and 9-nitro camptothecin (Rubitecan) that are poorly soluble in both aqueous and nonaqueous media and have been tested in humans. 9-nitro camptothecin is a prodrug of 9-amino camptothecin and spontaneously converts to 9-amino camptothecin in aqueous media and in vivo in mice, dogs and humans (Hinz et al., Pharmacokinetics of the in vivo and in vitro Conversion of 9-Nitro-20(S)-camptothecin to 9-Amino-20(S)-camptothecin in Humans, Dogs and Mice, Cancer Research 54:3096-3100; 1994).

The pharmacokinetic behavior of 9-nitro camptothecin and 9-amino camptothecin is similar to the water soluble camptothecin derivatives (Topotecan and Irinotecan) in that the plasma half lives are much shorter than the more lipid soluble CPT derivatives. Another major problem with 9-amino camptothecin is that its chemical synthesis using the semisynthetic method is carried out by nitration of CPT, followed by reduction to the amino group, which is a low yield synthesis. In addition, 9-amino camptothecin is light sensitive, heat sensitive and oxygen sensitive which renders the production and stabilization of 9-amino camptothecin difficult. The chemical decomposition reactions of 9-amino camptothecin can result in the formation of compounds that exhibit a large degree of toxicity in nude mice, whereas pure 9-amino camptothecin is significantly less toxic.

9-amino camptothecin is also difficult to administer to patients because it is poorly soluble in both aqueous and organic solvents. 9-nitro camptothecin is easier to produce and is more chemically stable, but with the chemical conversion to 9-amino camptothecin the drug is reportedly susceptible to MDR/MRP mediated drug resistance, which further limits its utility in the unfortunately common setting of drug resistant neoplasms. Based on pharmacokinetic behavior and chemical properties, 9-amino camptothecin is predicted to have reduced tissue penetration and retention relative to more lipid soluble camptothecin derivatives. Further, its poor solubility diminishes the amount of the drug that can cross the blood/brain barrier.

Of this diverse group of substituted CPT derivatives undergoing human clinical development, Irinotecan (CPT-11) has been one of the most extensively studied in Phase I and Phase II clinical trials in human patients with cancer. It is noteworthy that Irinotecan, which is a water soluble prodrug, is biologically inactive and requires activation by a putative carboxylesterase enzyme. The active species of Irinotecan is the depiperidenylated 10-hydroxy-7-ethyl camptothecin (claimed in Miyasaka et al. U.S. Patent # 4,473,692 (1984)), which is also known as SN38. SN38 is a toxic lipophilic metabolite, which is formed by an *in vivo* bioactivation of Irinotecan by a putative carboxylesterase enzyme.

SN38 is very poorly soluble in water and has not been directly administered to human patients with cancer. Recently, it has been reported in human patients that SN38 undergoes further metabolism to form a glucuronide species, which is an inactive form of the drug with respect to antitumor activity, and also appears to be involved in producing human toxicity (diarrhea, leukopenia) and substantial interpatient variability in drug levels of the free metabolite and its glucuronide.

Irinotecan has been tested in human clinical trials in the United States, Europe and Japan. Nearly 100 patient deaths directly attributable to Irinotecan drug toxicity have been reported in Japan alone. The Miyasaka et al. patents (U.S. Patent # 4,473,692 and 4,604,463) state that the object of their invention is to "provide 10-substituted camptothecins which are strong in anti-tumor activity and possess good absorbability in living bodies with very low toxicity" and "to provide new camptothecin derivatives which are strong in anti-tumor activity and possess good solubility in water and an extremely low toxicity".

Having multiple drug-related human deaths and serious patient toxicity, is clearly a failure of the Miyasaka et al. inventions to fulfill their stated objects. It is notable that tremendous interpatient variability with regard to drug levels of various forms, drug metabolism, certain pharmacokinetic properties and toxicity has been reported with the use of Irinotecan in human subjects with cancer. Parenteral administration of Irinotecan can achieve micromolar plasma concentrations of Irinotecan that, through metabolism to form SN38, can yield nanomolar concentrations of the active metabolite SN38. It has recently been reported in human subjects that SN38 undergoes further metabolism to form the SN38 glucuronide (Gupta et al. Metabolic Fate of Irinotecan in Humans: Correlation of Glucuronidation with Diarrhea. Cancer Research 54:3723-3725).

This further metabolic conversion of Irinotecan is important, since there is also reportedly large variability in the conversion of Irinotecan to SN38 and large interpatient variability in the metabolism of SN38 to form the inactive (and toxic) SN38 glucuronide in human subjects. (Gupta et al. Metabolic Fate of Irinotecan in Humans: Correlation of Glucuronidation with Diarrhea. Cancer Research 54:3723-3725; 1994 and Ohe, Y. et al., Phase I Study and Pharmacokinetics of CPT-11 with 5-Day Continuous Infusion. JNCI 84(12):972-974, 1992).

Since the amount of Irinotecan and SN38 metabolized is not predictable in individual patients, significant clinical limitations are posed and create the risk of life-threatening drug toxicity, and/or risk of drug inactivity due to five possible mechanisms: (1) conversion of greater amounts of Irinotecan to SN38; (2) inactivation of SN38 by glucuronidation; (3) conversion of SN38 glucuronide to free SN38; (4) lack of antineoplastic activity due to the conversion of lesser amounts of Irinotecan to form SN38; and (5) lack of antineoplastic activity by more rapid and extensive conversion of SN38 to form the glucuronide species. It is important to note that even a doubling of the plasma concentration of the potent Irinotecan metabolite SN38 may result in significant toxicity, because free SN38 exhibits antineoplastic activity at nanomolar concentrations.

Another source of interpatient variability and toxicity is the *in vivo* de-glucuronidation of SN38 and similar CPT derivatives to produce a free and active species of the drug. Deglucuronidation of a CPT derivative that is susceptible to A-ring glucuronidation, such as SN38, results in an increase in the plasma or local tissue concentration of the free and active form of the drug, and if high enough levels were reached, patient toxicity, and even death may result.

In addition to the two approved drugs, there are currently at least nine camptothecin derivatives in various stages of human clinical trials:

### 1. Karenitecin (BNP1350)

BNP1350 is a highly lipophilic camptothecin derivative having a 7-trimethylsilylethyl moiety. Claimed in United States Patent 5,910,491, along with formulations and uses thereof. Formulations of BNP1350 with N-methylpyrrolidinone (NMP) are claimed in United States Patent 5,726,181 and others. WO 98/35940 A1 discloses 7-(β trimethylsilyl)ethyl camptothecin.

### 2. Lurtotecan (NX 211)

NX211 is a water-soluble camptothecin having a 10,11-ethylenedioxy moiety and a cleavable 4-methyl piperazino methyl moiety at C7. United States Patent 5,559,235 and others describes and claims the compound, and formulations and uses thereof.

### 3. Exatecan (DX-8951f)

DX-8951f is a hexacyclic camptothecin derivative, having 10-methyl and 11-fluoro substitutions, and with its sixth ring fused between C7 and C9. United States Patent 5,637,770 and others describes and claims the compound, and formulations and uses thereof.

### 4. Diflomotecan (BN 80915)

### 5. BN 80915 is a 10,11 difluoro camptothecin, with a 7-member E-ring (homocamptothecin).

United States Patent 5,981,542 and others describes and claims the compound, and its uses and formulations.Rubitecan (9-Nitro CPT) 9-nitro camptothecin, as mentioned above is poorly soluble in both aqueous and organic solvents and is described and is not claimed any United States Patents, with the first publication of the compound occurring in Japanese Patent Application No. 82-160944 in 1982. Several patents have issued since then, all regarding processes for preparing the compound as well as uses thereof.

### 6. Afeletecan (CPT Glycoconjugate)

Afeletecan is an C20 glycoconjugated, water-soluble derivative of camptothecin and is described and claimed in United States Patent 6,492,335.

### 7. Gimatecan (ST 1481)

ST1481 is a water-soluble prodrug having a C7 imino moiety, bonded to a terminal *tert-*butoxy group. The compound is described and claimed in United States Patent 6,242,257.

### 8. Mureletecan (PNU 166148)

Mureletecan is another water-soluble prodrug having a cleavable peptide moiety bonded to C20 to form an ester.

### 9. Pegbetotecan, Pegcamotecan, Peglinxotecan (PEG CPT; Prothecan®)

This prodrug includes a cleavable water-soluble polyethylene glycol moiety that forms an ester at C20. The compound is described and claimed in United States Patent 5,840,900 and others.

The structures of the above molecules are set forth below in Table 1.

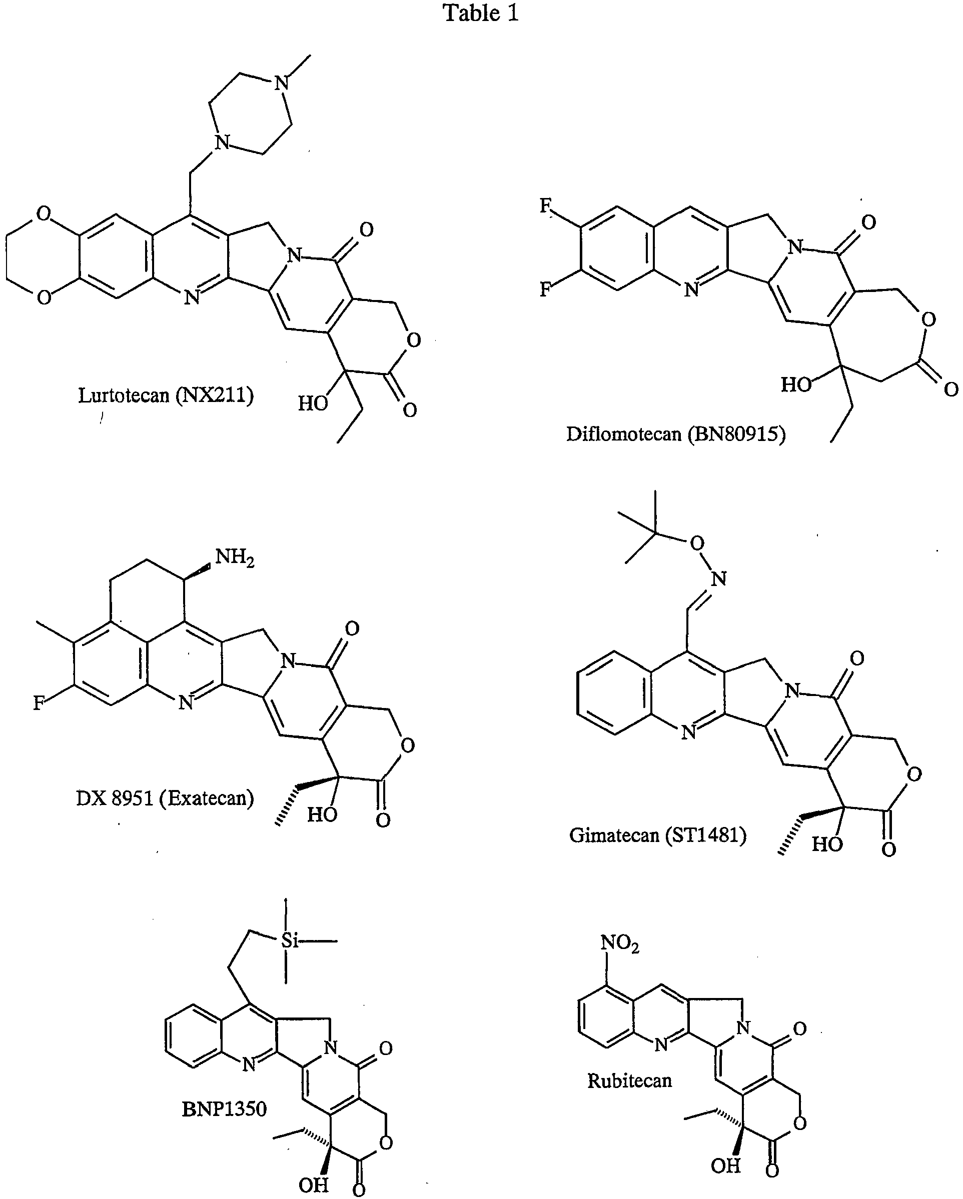

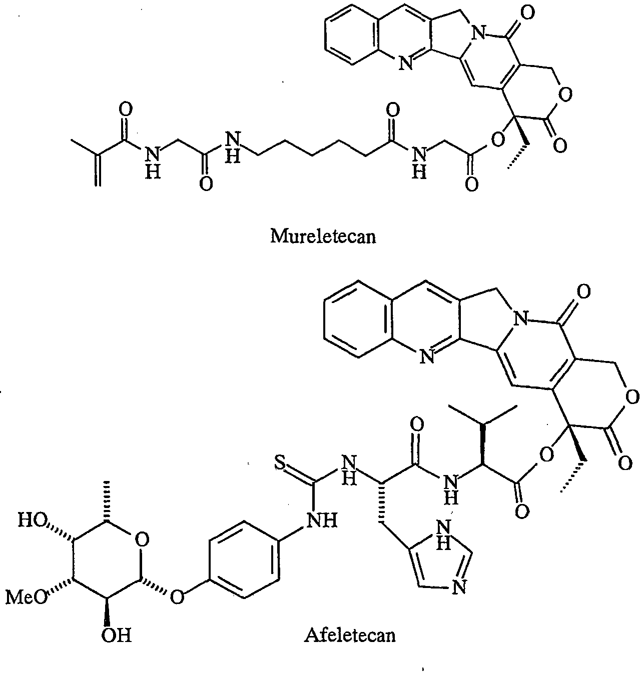

Poorly water-soluble camptothecins are necessarily formulated for administration by dissolution or suspension in organic solvents. United States Patents 5,447,936; 5,726,181; 5,859,022; 5,859,023; 5,880,133; 5,900,419; 5,935,967; 5,955,467; and other describe pharmaceutical formulations of highly lipophilic, poorly water-soluble camptothecin derivatives in various organic solvents, namely N,N-dimethylacetamide (DMA); N,N-dimethylisosorbide (DMI); and N-methylpyrrolidinone (NMP).

### SUMMARY OF THE INVENTION

This invention relates a process for preparing a pharmaceutical formulation adapted for administration to human patients, said formulation comprising an effective amount of medicinal grade 7-(2-trimethylsilylethyl) camptothecin dissolved in a solution consisting essentially of:
a) 10% to 20% by weight of one or more solvents selected from N-methylpyrrolidone and N,N-dimethylacetamide;
b) 5% to 25% by weight of a non-ionic surfactant;
c) 1% to 10% by weight of a low molecular weight alcohol;
d) 50% to 80% by weight of a low molecular weight polyethylene glycol; and
e) 0.1 % to 2.0% by weight of a pharmaceutically acceptable acid,
the process comprising the following steps:
a) weighing the desired amount of the low molecular weight alcohol and adding to a first compounding vessel;
b) weighing the desired amount of the pharmaceutically acceptable acid, adding to the first compounding vessel, and mixing until the acid is completely dissolved;
c) adding the desired amount of solvent to a second compounding vessel;
d) weighing the desired amount of the 7-(2-trimethylsilylethyl) camptothecin and adding to the second compounding vessel;
e) mixing the contents of the second compounding vessel until the 7-(2-trimethylsilylethyl) camptothecin is dispersed and heating the second compounding vessel to a temperature of 30°C to 60°C;
f) sonicating the contents of the second compounding vessel until the 7-(2-trimethylsilylethyl) camptothecin is dissolved;
g) adding the non-ionic surfactant and the low molecular weight polyethylene glycol to the first compounding vessel and heating to a temperature of 30°C to 60°C; and
h) transferring the contents of the second compounding vessel to the first compounding vessel while maintaining heating to a temperature of 30°C to 60°C, and mixing until a homogenous solution is formed.
The formulation is adapted for administration by intravenous route to human patients as treatment for various solid tumors.

After dissolution, the formulation is preferably packaged in unit dose form for parenteral administration to a patient undergoing treatment for cancer or other disease that the active ingredient is used to treat.

Like most oncology agents, the therapeutic index for the active ingredient is narrow, and requires a precisely measured dose to be delivered.

The contents of the standby vessel are used to rinse the second compounding vessel to ensure complete transfer of its contents to the first compounding vessel.

Additionally, the process may also include steps of filtering the final product, and filling the filtered solution into unit dose vessels for administration to the patient. Preferred formulations are disclosed in the specification below, and do not limit the scope of the invention, which is defined by the claims.

### DESCRIPTION OF THE PREFERRED EMBODIMENTS

The preferred embodiments herein described are not intended to be exhaustive or to limit the invention to the precise details set forth below. They are chosen and described to be illustrative of the invention, and to explain the principles of the invention and their application and practical use to those skilled in the art.

Also disclosed is a formulation which is particularly suitable for administration to human patients. The formulation is most preferably adapted for intravenous administration, but may be administered by any convenient parenteral route. The formulation includes as its active ingredient a highly lipophilic camptothecin derivative (HLCD) as that term is recognized in the art. HLCDs are defined as having a water solubility of less than 5 micrograms per milliliter of water. The HLCD is preferably of medicinal grade suitable for administration to human patients. For purposes of this application, medicinal grade means the HLCD is at least 98% pure.

The formulation also includes the following ingredients:
a) 10% to 20% by weight of one or more solvents;
b) 5% to 25% by weight of a nonionic surfactant;
c) 1% to 10% by weight of a low molecular weight alcohol;
d) 50% to 80% by weight of a low molecular weight polyethylene glycol; and
e) 0.1% to 2.0% by weight of a pharmaceutically acceptable acid.

The formulation is prepared according to the following general process:
a. Weighing the desired amount of the low molecular weight alcohol and adding to a first compounding vessel;
b. Weighing the desired amount of the pharmaceutically acceptable acid, adding to the first compounding vessel, and mixing until the acid is completely dissolved;
c. Adding the desired amount of solvent to a second compounding vessel;
d. Weighing the desired amount of the highly lipophilic camptothecin derivative and adding to the second compounding vessel;
e. Mixing the contents of the second compounding vessel until the highly lipophilic camptothecin derivative is dispersed and heating the second compounding vessel to between 30 °C to 60 °C;
f. Sonicating the contents of the second compounding vessel until the highly lipophilic camptothecin derivative is dissolved;
g. Adding the nonionic surfactant and the low molecular weight polyethylene glycol to the first compounding vessel and heating to between 30 °C to 60 °C; and
h. Transferring the contents of the second compounding vessel to the first compounding vessel while maintaining heating to between 30 °C to 60 °C, and mixing until a homogenous solution is formed.

The HLCD is 7-(2-trimethylsilylethyl) camptothecin (BNP1350; Karenitecin). The solvents are N-methylpyrrolidinone (NMP) or dimethylacetamide (DMA) or a combination of two of the above may be used as co-solvents. The most preferred solvent is NMP, or a combination of NMP and DMA as co-solvents.

Preferred surfactants include polysorbates; sorbitan esters; nonoxynols; and others, the most preferred surfactant being polysorbate 80. Preferred alcohols include ethyl alcohol and benzyl alcohol, most preferred is ethyl alcohol. The preferred low molecular weight polyethylene glycol (PEG) includes PEG 100, PEG 200, PEG 300, PEG 400, PEG 600, PEG 800, and the most preferred is PEG 300.

The formulation is filtered and purified following the combining of the ingredients, then dispensed into sterile unit dose containers. The containers are of a color to protect the contents from light, and are sealed with a sterile seal following filling. One or more labels are affixed to the unit dose container to identify the contents of the container, and/or provide instructions for administration and safety procedures regarding the formulation contained therein.

The following examples are illustrative of the processes employed to produce the formulation of this invention.

### Example 1

### Bulk Formulation

| Material | Amount |
|---|---|
| Citric Acid, Anhydrous, USP | 400.0 g |
| Dehydrated Alcohol, USP | 2559.0 mL |
| N-methylpyrrolidinone (NMP) | 4143.3 g |
| BNP1350 (at least 98% pure) | 2800.0 mg |
| Polysorbate-80, NF | 4000.0 g |
| PEG-300 NF | Qs to 30,240.0 g |

### Equipment

40L Carboy, 9L Carboy, 4L Graduated Cylinder, Lightnin' Labmaster Mixer with Teflon coated impeller and stir rod, Heat Belts, Water Bath with sonication capability

### Procedures

All facilities and equipment is verified clean and suitable for use in making pharmaceutical preparations. The 40L carboy is weighed and set aside. 2559.0 mL of USP grade dehydrated alcohol is measured into the 4L graduated cylinder and an agitator is placed in the cylinder and activated. 400.0 g of citric acid anhydrous, USP grade is weighed and added to the dehydrated alcohol. The cylinder is covered to prevent evaporation of the alcohol and stirring is continued until the acid is completely dissolved. If necessary, the mixture may be heated to 25-30 °C to aid dissolution, and is then allowed to cool to room temperature. The carboy and solution are weighed and the weight recorded.

4143.3 g of NMP is added to the 9L carboy. 500-600 mL is withdrawn to a separate vessel for later use as a rinse. 2800.0 mg of BNP1350 is weighed and added to the 9L carboy. The shaft of the Labmaster mixer is positioned in the carboy and started, rotating at 500 rpm for at least 10 minutes to disperse the BNP1350 in the NMP. Speed is reduced if the solution begins to foam. Heat belts and water bath are used to warm the mixture to between 45-50 °C and the mixture is sonicated for at least 30 minutes, or until no particulate matter is visible. The Labmaster shaft is then withdrawn from the carboy.

After sonication, the NMP/BNP1350 mixture is added to the 40L carboy, which contains the alcohol and acid solution. The 9L carboy is rinsed with the withdrawn NMP, and the contents added to the 40L carboy.

4000.0 g of polysorbate-80, NF is then weighed into the 40L carboy. PEG-300 is added to the carboy until the total solution weight is 30,240.0 g and the Labmaster shaft is reinserted and started at slow speed. The solution is mixed thoroughly for at least 60 minutes at slow speed to prevent frothing of the solution.

The solution is visually checked for clarity and completeness of solution and a 5 mL sample is withdrawn and assayed for concentration of BNP1350 (0.1 mg/mL ± 0.005) prior to filtering.

### Example 2

### Filtering and Filling Bulk Formulation Into Unit Dose Containers

### Equipment

Pall HDC II 0.6 micron Abs sterile prefilter
Pall Sol-vent DCF capsule 0.2 micron sterile filter
5 mL amber fill bottles, 20 mm opening- sterile
20 mm Teflon^{®} 4432/50 gray stoppers- sterile
20 mm white flip-off seals- sterile

### Sterile filtration tubing

### Procedures

All facilities and equipment are verified to be clean and suitable for use in the filter and filling operations for pharmaceutical products. After the facilities (hereafter, clean room) are verified to be suitably sterile, the solution from Example 1 is transferred to the clean room. The clean room is continuously monitored for airborne particles and viable flora as well as pressure differential compared to the pressure outside the clean room. A heat belt is applied to the 40L carboy containing the Example 1 solution to warm the solution to between 35-40 °C. After the solution has been allowed to sit at this temperature overnight, the solution is filtered through the 0.6 micron sterile prefilter and then through the 0.2 micron sterile filter. When the solution has all passed through the filters, the filter is then bubble tested and flushed using a solution of 60% isopropyl alcohol and 40% water and the water bubble point of 11 psi should be reached. If the test fails, filtration must be repeated with new filters until a successful bubble test is obtaineu. A 5 mL sample of the solution is withdrawn and assayed for purity prior to proceeding to the filling step.

The solution is then transferred to a Flexicon^{®} semi-automatic filling machine, which dispenses 6.0 g ± 0.1 g into each sterile vial. A sterile stopper is then applied to each vial and finally a sterile seal is applied and crimped to each vial. 20-40 vials are removed for testing after filling and sealing. The number of vials filled is recorded and the vials transferred to a quarantine area for inspection. After the vials are inspected, a label having printed information regarding the contents, instructions for use and/or safety information is affixed to each vial. The concentration of BNP1350 contained in each vial is 0.1 mg/mL, which is labeled for use as an antineoplastic agent for injection.

### Example 3

### Bulk Formulation

| Material | Amount |
|---|---|
| Citric Acid, Anhydrous, USP | 127.92 g |
| Dehydrated Alcohol, USP | 644.40 g |
| N-methylpyrrolidinone (NMP) | 894.72 g |
| N,N-dimethylacetamide (DMA; Omnisolve) | 1150.44 g |
| BNP1350 (at least 98% pure) | 1200.0 mg |
| Polysorbate-80, NF | 1662.12 g |
| PEG-300 NF | 8307.36 g |

### Equipment

### 13L Carboy, 100 mL Beaker, 4L Flask, Lightnin' Labmaster Mixer, Heat Belts, Water Bath Procedures

All facilities and equipment is verified clean and suitable for use in making pharmaceutical preparations. The 13L carboy is weighed and set aside. 644.40 g of USP grade dehydrated alcohol is weighed and poured into the 4L flask and a magnetic stirrer is placed in the cylinder and activated. 127.92 g of citric acid anhydrous, USP grade is weighed and added to the dehydrated alcohol. The cylinder is covered to prevent evaporation of the alcohol and stirring is continued until the acid is completely dissolved. If necessary, the mixture may be heated to 25-30 °C to aid dissolution, and is then allowed to cool to room temperature. The flask and solution are weighed and the weight recorded.

894.72 g of NMP and 1150.44 g of DMA is added to the alcohol/citric acid solution and mixed for at least 10 minutes to form a homogenous solution. About ¾ of the solution is withdrawn to a separate vessel for later use as a rinse. 1200.0 mg of BNP1350 is weighed and added to the 100 mL beaker and then added to the 4L flask. The beaker is rinsed with portions of the withdrawn solution noted above and the washings added to the 4L flask. The shaft of the Labmaster mixer is positioned in the flask and started, rotating at 1000 rpm for at least 10 minutes to disperse the BNP1350. Speed is reduced if the solution begins to foam. Heat belts and water bath are used to warm the mixture to between 45-50 °C and the mixture is sonicated for at least 30 minutes, or until no particulate matter is visible. The Labmaster shaft is then withdrawn from the flask.

1662.12 g of polysorbate-80, NF is then weighed into the 13L carboy. 8307.36 g of PEG-300 is added to the carboy and the carboy is placed on a magnetic stirrer and stirred at medium speed. Heat belts are applied to the carboy to raise the temperature to between 45-50 °C. After the carboy has reached this temperature, the contents of the 4L flask are transferred to the 13L carboy. The remainder of the withdrawn solution is used to rinse the 4L flask, and the contents transferred to the 13L carboy. The carboy is maintaining at 45-50 °C for a minimum of 60 minutes and the stirrer speed watched to ensure that the solution does not froth.

The solution is visually checked for clarity and completeness of solution and a 5 mL sample is withdrawn and assayed for concentration (0.1 ± 0.005 mg/mL) prior to filtering.

### Example 4

### Filtering and Filling Bulk Formulation Into Unit Dose Containers

### Equipment

Pall HDC II 0.6 micron Abs sterile prefilter
Pall Sol-vent DCF capsule 0.2 micron sterile filter
5 mL amber fill bottles, 20 mm opening- sterile
20 mm Teflon^{®} 4432/50 gray stoppers- sterile
20 mm white flip-off seals- sterile

### Sterile filtration tubing

### Procedures

All facilities and equipment are verified to be clean and suitable for use in the filter and filling operations for pharmaceutical products. After the facilities (hereafter, clean room) are verified to be suitably sterile, the solution from Example 1 is transferred to the clean room. The clean room is continuously monitored for airborne particles and viable flora as well as pressure differential compared to the pressure outside the clean room. A heat belt is applied to the 40L carboy containing the Example 1 solution to warm the solution to between 35-40 °C. After the solution has been allowed to sit at this temperature overnight, the solution is filtered through the 0.6 micron sterile prefilter and then through the 0.2 micron sterile filter. When the solution has all passed through the filters, the filter is then bubble tested and flushed using a solution of 60% isopropyl alcohol and 40% water and the water bubble point of 11 psi should be reached. If the test fails, filtration must be repeated with new filters until a successful bubble test is obtained. A 5 mL sample of the solution is withdrawn and assayed for purity prior to proceeding to the filling step.

The solution is then transferred to a Flexicon^{®} filling machine, which dispenses 6.0 g ± 0.1 g into each sterile vial. A sterile stopper is then applied to each vial and finally a sterile seal is applied and crimped to each vial. 20-40 vials are removed for testing after filling and sealing. The number of vials filled is recorded and the vials transferred to a quarantine area for inspection. After the vials are inspected, a label having printed information regarding the contents, instructions for use and/or safety information is affixed to each vial. The concentration of BNP1350 contained in each vial is 0.1 mg/mL, which is labeled for use as an antineoplastic agent for injection.

## Claims

1. A process for preparing a pharmaceutical formulation adapted for administration to human patients, said formulation comprising an effective amount of medicinal grade 7-(2-trimethylsilylethyl) camptothecin dissolved in a solution consisting essentially of:
a) 10% to 20% by weight of one or more solvents selected from N-methylpyrrolidone and N,N-dimethylacetamide;
b) 5% to 25% by weight of a non-ionic surfactant;
c) 1% to 10% by weight of a low molecular weight alcohol;
d) 50% to 80% by weight of a low molecular weight polyethylene glycol; and
e) 0.1 % to 2.0% by weight of a pharmaceutically acceptable acid,
the process comprising the following steps:
a) weighing the desired amount of the low molecular weight alcohol and adding to a first compounding vessel;
b) weighing the desired amount of the pharmaceutically acceptable acid, adding to the first compounding vessel, and mixing until the acid is completely dissolved;
c) adding the desired amount of solvent to a second compounding vessel;
d) weighing the desired amount of the 7-(2-trimethylsilylethyl) camptothecin and adding to the second compounding vessel;
e) mixing the contents of the second compounding vessel until the 7-(2-trimethylsilylethyl) camptothecin is dispersed and heating the second compounding vessel to a temperature of 30°C to 60°C;
f) sonicating the contents of the second compounding vessel until the 7-(2-trimethylsilylethyl) camptothecin is dissolved;
g) adding the non-ionic surfactant and the low molecular weight polyethylene glycol to the first compounding vessel and heating to a temperature of 30°C to 60°C; and
h) transferring the contents of the second compounding vessel to the first compounding vessel while maintaining heating to a temperature of 30°C to 60°C, and mixing until a homogenous solution is formed.

2. The process of claim 1 wherein the process includes the additional steps of:
i) filtering the homogenous solution from step h) to remove particulate therefrom;
j) providing a sterile vial, and dispensing a predetermined amount of the filtered solution into said sterile vial; and
k) sealing said sterile vial with a sterile seal.

3. The process of claim 1 or 2, wherein the process includes the additional steps c1) transferring 25% to 90% of the solvent to a standby vessel; and h1) rinsing the second compounding vessel with the contents of the standby vessel, and then again transferring the contents of the second compounding vessel to the first compounding vessel.

4. The process of claim 1 wherein the effective amount of 7-(2-trimethylsilylethyl) camptothecin is from 0.01 mg/mL to 0.5 mg/mL.

5. The process of claim 1 wherein the non-ionic surfactant is polysorbate 80.

6. The process of claim 1 wherein the pharmaceutically acceptable acid is citric acid.

7. The process of claim 1 wherein the low molecular weight alcohol is ethanol.

8. The process of claim 1 wherein N-methylpyrrolidone and N,N-dimethylacetamide are co-solvents, each contained at 5% to 15% by weight of the solution.

9. The process of claim 1 wherein the formulation is dissolved in a pharmaceutically acceptable diluent prior to administration.

10. The process of claim 9 wherein the diluent is 5% dextrose solution.

11. The process of claim 9 wherein the diluent is 0.9% sodium chloride solution.

12. The process of claim 1 wherein the formulation consists essentially of:
a) 0.01 mg/mL to 0.50 mg/mL of medicinal grade 7-(2-trimethylsilylethyl) camptothecin;
b) 10% to 20% by weight of N-methylpyrrolidinone;
c) 5% to 25% by weight of polysorbate 80;
d) 1.0% to 10.0% by weight of ethyl alcohol;
e) 50% to 80% by weight of PEG 300; and
f) 0.1 % to 2.0% by weight of citric acid.

13. The process of claim 12 wherein the ethyl alcohol is dehydrated ethyl alcohol.

14. The process of claim 12 wherein the formulation consists essentially of:
a) 0.05 to 0.2 mg/mL of substantially pure 7-(2-trimethylsilylethyl) camptothecin;
b) 13% to 14% by weight of N-methylpyrrolidinone;
c) 13% to 14% by weight of polysorbate 80;
d) 64% to 66% by weight of PEG 300;
e) 6% to 7% by weight of dehydrated ethyl alcohol; and
f) 1.0% to 1.4% by weight of citric acid.

15. The process of claim 14 wherein the formulation is stored in a sterile unit dose container that is aseptically sealed and protected from light.

16. The process of claim 15 wherein prior to administration to a patient the formulation is diluted in a pharmaceutically acceptable diluent.

## Patentansprüche

1. Verfahren zur Herstellung einer pharmazeutischen Formulierung angepasst zur Verabreichung an menschliche Patienten, die Formulierung umfasst eine effektive Menge von 7-(2-Trimethylsilylethyl)-camptothecin, pharmazeutischer Qualität, gelöst in einer Lösung, welche im Wesentlichen aus:
a) 10 bis 20 Gewichtsprozent von einem oder mehreren Lösungsmitteln ausgewählt aus N-Methylpyrrolidon und N,N-Dimethylacetamid;
b) 5 bis 25 Gewichtsprozent eines nicht-ionischen Tensids;
c) 1 bis 10 Gewichtsprozent eines niedermolekularen Alkohols;
d) 50 bis 80 Gewichtsprozent eines niedermolekularen Polyethylenglykols; und
e) 0,1 bis 2,0 Gewichtsprozent einer pharmazeutisch akzeptablen Säure, besteht,
wobei das Verfahren die folgenden Schritte umfasst:
a) Abwiegen der gewünschten Menge des niedermolekularen Alkohols und Hinzufügen in ein erstes Kompoundierungsbehältnis;
b) Abwiegen der gewünschten Menge der pharmazeutisch akzeptablen Säure, Hinzufügen in das erste Kompoundierungsbehältnis und Mischen bis die Säure vollständig gelöst ist;
c) Hinzufügen der gewünschten Menge von Lösungsmittel in ein zweites Kompoundierungsbehältnis;
d) Abwiegen der gewünschten Menge des 7-(2-Trimethylsilylethyl)-camptothecins und Hinzufügen in das zweite Kompoundierungsbehältnis;
e) Mischen des Inhalts des zweiten Kompoundierungsbehältnisses bis das 7-(2-Trimethylsilylethyl)-camptothecin dispergiert ist und Heizen des zweiten Kompoundierungsbehältnisses auf eine Temperatur von 30°C bis 60°C;
f) Ultraschallbehandlung der Inhalte des zweiten Kompoundierungsbehältnisses bis das 7-(2-Trimethylsilylethyl)-camptothecin gelöst ist;
g) Hinzufügen des nicht-ionischen Tensids und des niedermolekularen Polyethyleneglykols in das erste Kompoundierungsbehältnis und Heizen auf eine Temperatur von 30°C bis 60°C; und
h) Überführen der Inhalte des zweiten Kompoundierungsbehältnisses in das erste Kompoundierungsbehältnis unter Beibehaltung des Heizens auf eine Temperatur von 30°C bis 60°C und Mischen bis eine homogene Lösung gebildet ist.

2. Das Verfahren von Anspruch 1, wobei das Verfahren die zusätzlichen Schritte von:
i) Filtern der homogenen Lösung aus Schritt h), um Partikel davon zu entfernen;
j) Bereitstellen eines sterilen Gefäßes und Dosieren einer vorbestimmten Menge der gefilterten Lösung in das sterile Gefäß; und
k) Verschließen des sterilen Gefäßes mit einem sterilen Verschluss; beinhaltet.

3. Das Verfahren von Anspruch 1 oder 2, wobei das Verfahren die zusätzlichen Schritte c1) Überführen von 25% bis 90% des Lösungsmittels in ein Standby-Behältnis; und h1) Durchspülen des zweiten Kompoundierungsbehältnisses mit den Inhalten des Standby-Behältnisses und dann erneut Überführen der Inhalte des zweiten Kompoundierungsbehältnisses in das erste Kompoundierungsbehältnis beinhaltet.

4. Das Verfahren von Anspruch 1, wobei die effektive Menge von 7-(2-Trimethylsilylethyl)-camptothecin von 0,01 mg/mL bis 0,5 mg/mL ist.

5. Das Verfahren von Anspruch 1, wobei das nicht-ionische Tensid Polysorbat 80 ist.

6. Das Verfahren von Anspruch 1, wobei die pharmazeutisch akzeptable Säure Zitronensäure ist.

7. Das Verfahren von Anspruch 1, wobei der niedermolekulare Alkohol Ethanol ist.

8. Das Verfahren von Anspruch 1, wobei N-Methylpyrrolidon und N,N-Dimethylacetamid Co-Lösungsmittel sind, jedes zu 5 bis 15 Gewichtsprozent der Lösung enthalten.

9. Das Verfahren von Anspruch 1, wobei die Formulierung in einem pharmazeutisch akzeptablen Verdünnungsmittel vor der Verabreichung gelöst wird.

10. Das Verfahren von Anspruch 9, wobei das Verdünnungsmittel 5% Dextroselösung ist.

11. Das Verfahren von Anspruch 9, wobei das Verdünnungsmittel 0,9% Natriumchloridlösung ist.

12. Das Verfahren von Anspruch 1, wobei die Formulierung im Wesentlichen aus:
a) 0,01 mg/mL bis 0,50 mg/mL 7-(2-Trimethylsilylethyl)-camptothecin, medizinischer Qualität;
b) 10 bis 20 Gewichtsprozent N-Methylpyrrolidinon;
c) 5 bis 25 Gewichtsprozent Polysorbat 80;
d) 1,0 bis 10,0 Gewichtsprozent Ethylalkohol;
e) 50 bis 80 Gewichtsprozent PEG 300; und
f) 0,1 bis 2,0 Gewichtsprozent Zitronensäure,
besteht.

13. Das Verfahren von Anspruch 12, wobei der Ethylalkohol wasserfreier Ethylalkohol ist.

14. Das Verfahren von Anspruch 12, wobei die Formulierung im Wesentlichen aus:
a) 0,05 bis 0,2 mg/mL von im Wesentlichen reinen 7-(2-Trimethylsilylethyl)-camptothecin;
b) 13 bis 14 Gewichtsprozent N-Methylpyrrolidinon;
c) 13 bis 14 Gewichtsprozent Polysorbat 80;
d) 64 bis 66 Gewichtsprozent PEG 300;
e) 6 bis 7 Gewichtsprozent wasserfreiem Ethylalkohol; und
f) 1,0 bis 1,4 Gewichtsprozent Zitronensäure,
besteht.

15. Das Verfahren von Anspruch 14, wobei die Formulierung in einem sterilen Einheitendosierungsbehälter, der aseptisch versiegelt und vor Licht geschützt ist, aufbewahrt wird.

16. Das Verfahren von Anspruch 15, wobei vor Verabreichung an einen Patienten die Formulierung in einem pharmazeutisch akzeptablen Verdünnungsmittel verdünnt wird.

## Revendications

1. Procédé de préparation d'une formulation pharmaceutique adaptée pour une administration à des patients humains, ladite formulation comprenant une quantité efficace de 7-(2-triméthylsilyléthyl)camptothécine de qualité médicinale dissoute dans une solution consistant essentiellement en :
a) 10 % à 20 % en poids d'un ou plusieurs solvants choisis parmi la N-méthylpyrrolidone et le N,N-diméthylacétamide ;
b) 5 % à 25 % en poids d'un tensioactif non ionique ;
c) 1 % à 10 % en poids d'un alcool de faible masse moléculaire ;
d) 50 % à 80 % en poids d'un poly(éthylène glycol) de faible masse moléculaire ; et
e) 0,1 % à 2,0 % en poids d'un acide pharmaceutiquement acceptable,
le procédé comprenant les étapes suivantes consistant à :
a) peser la quantité souhaitée de l'alcool de faible masse moléculaire et l'ajouter à un premier réservoir mélangeur ;
b) peser la quantité souhaitée de l'acide pharmaceutiquement acceptable, l'ajouter à un premier réservoir mélangeur et mélanger jusqu'à ce que l'acide soit complètement dissous ;
c) ajouter la quantité souhaitée de solvant à un second réservoir mélangeur ;
d) peser la quantité souhaitée de la 7-(2-triméthylsilyléthyl)camptothécine et l'ajouter au second réservoir mélangeur ;
e) mélanger le contenu du second réservoir mélangeur jusqu'à ce que la 7-(2-triméthylsilyléthyl)camptothécine soit dispersée et chauffer le second réservoir mélangeur à une température de 30 °C à 60 °C ;
f) soniquer le contenu du second réservoir mélangeur jusqu'à ce que la 7-(2-triméthylsilyléthyl)camptothécine soit dissoute ;
g) ajouter le tensioactif non ionique et le poly(éthylène glycol) à faible masse moléculaire au premier réservoir mélangeur et chauffer à une température de 30 °C à 60 °C ; et
h) transférer le contenu du second réservoir mélangeur dans le premier réservoir mélangeur tout en maintenant le chauffage à une température de 30 °C à 60 °C, et mélanger jusqu'à ce qu'une solution homogène soit formée.

2. Procédé selon la revendication 1, dans lequel le procédé inclut les étapes additionnelles consistant à :
i) filtrer la solution homogène de l'étape h) pour en enlever les particules ;
j) fournir un flacon stérile, et distribuer une quantité prédéterminée de la solution filtrée dans ledit flacon stérile ; et
k) obturer ledit flacon stérile avec un joint stérile.

3. Procédé selon la revendication 1 ou 2, dans lequel le procédé inclut les étapes additionnelles consistant à
c1) transférer 25 % à 90 % du solvant dans un réservoir d'attente ; et h1) rincer le second réservoir mélangeur avec le contenu du réservoir d'attente, puis à nouveau transférer le contenu du second réservoir mélangeur dans le premier réservoir mélangeur.

4. Procédé selon la revendication 1, dans lequel la quantité efficace de 7-(2-triméthylsilyléthyl)camptothécine est de 0,01 mg/mL à 0,5 mg/mL.

5. Procédé selon la revendication 1, dans lequel le tensioactif non ionique est le polysorbate 80.

6. Procédé selon la revendication 1, dans lequel l'acide pharmaceutiquement acceptable est l'acide citrique.

7. Procédé selon la revendication 1, dans lequel l'alcool de faible masse moléculaire est l'éthanol.

8. Procédé selon la revendication 1, dans lequel la N-méthylpyrrolidone et le N,N-diméthylacétamide sont des cosolvants, chacun contenu à hauteur de 5 % à 15 % en poids de la solution.

9. Procédé selon la revendication 1, dans lequel la formulation est dissoute dans un diluant pharmaceutiquement acceptable avant administration.

10. Procédé selon la revendication 9, dans lequel le diluant est une solution de dextrose à 5 %.

11. Procédé selon la revendication 9, dans lequel le diluant est une solution de chlorure de sodium à 0,9 %.

12. Procédé selon la revendication 1, dans lequel la formulation consiste essentiellement en :
a) 0,01 mg/mL à 0,50 mg/mL de 7-(2-triméthylsilyléthyl)camptothécine de qualité médicinale ;
b) 10 % à 20 % en poids de N-méthylpyrrolidinone ;
c) 5 % à 25 % en poids de polysorbate 80 ;
d) 1,0 % à 10,0 % en poids d'alcool éthylique ;
e) 50 % à 80 % en poids de PEG 300 ; et
f) 0,1 % à 2,0 % en poids d'acide citrique.

13. Procédé selon la revendication 12, dans lequel l'alcool éthylique est de l'alcool éthylique déshydraté.

14. Procédé selon la revendication 12, dans lequel la formulation consiste essentiellement en :
a) 0,05 à 0,2 mg/mL de 7-(2-triméthylsilyléthyl)camptothécine essentiellement pure ;
b) 13 % à 14 % en poids de N-méthylpyrrolidinone ;
c) 13 % à 14 % en poids de polysorbate 80 ;
d) 64 % à 66 % en poids de PEG 300 ;
e) 6 % à 7 % en poids d'alcool éthylique déshydraté ; et
f) 1,0 % à 1,4 % en poids d'acide citrique.

15. Procédé selon la revendication 14, dans lequel la formulation est stockée dans un contenant de dose unitaire stérile qui est obturé aseptiquement et protégé de la lumière.

16. Procédé selon la revendication 15, dans lequel avant administration à un patient, la formulation est diluée dans un diluant pharmaceutiquement acceptable.
